(19) Europäisches Patentamt
European Patent Office
Office européen des brevets

(11) Publication number: **0 052 238**
**B1**

(12) # EUROPEAN PATENT SPECIFICATION

(45) Date of publication of patent specification: **09.05.84**

(51) Int. Cl.[3]: **C 07 D 461/00,**
A 61 K 31/475

(21) Application number: **81108402.9**

(22) Date of filing: **16.10.81**

(54) **A new vincamine derivative, a method for its preparation and pharmaceutical compositions containing it.**

(30) Priority: **14.11.80 IT 2598280**

(43) Date of publication of application:
**26.05.82 Bulletin 82/21**

(45) Publication of the grant of the patent:
**09.05.84 Bulletin 84/19**

(84) Designated Contracting States:
**AT BE CH DE FR GB LI LU NL SE**

(56) References cited:
**FR - A - 2 275 209**
**GB - A - 1 405 127**
**GB - A - 1 445 956**

(73) Proprietor: **MEDEA RESEARCHES S.r.l.**
**Via Cappuccini, 20**
**I-20122 Milan (IT)**

(72) Inventor: **Quadro, Giuseppe**
**Via Pisacane, 34/A**
**Milan (IT)**

(74) Representative: **Bianchetti, Giuseppe**
**Studio Consulenza Brevettuale Via Senato 24**
**I-20121 Milan (IT)**

**The file contains technical information submitted after the application was filed and not included in this specification**

**EP 0 052 238 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention refers to a new vincamine derivative, to a method for its preparation and to pharmaceutical compositions containing it. More exactly, the new vincamine derivative which represents one of the objects of the present invention is the 13a-ethyl-2,3,5,6,13a,13b-hexahydro-1H-indolo[3,2,1-de]pyrido[3,2,1-ij]-[1,5]naphthyridine-12-methoxymethylcarboxylate of the following formula (I)

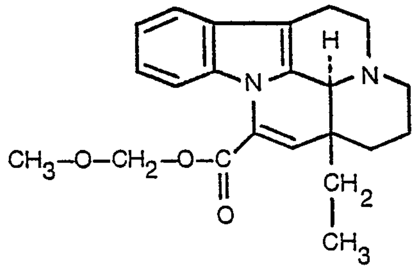

(I)

As it results from the above formula I, the compound according to the invention is a vincamine derivative. If compared with this latter compound, it possesses the following advantages:
—a lower acute toxicity
—a markedly superior vasodilating and myorelaxing activity.

Different kind of esters of apovincaminic acids are known: for instance, FR—A—2275209 describes esters with β-keto-alcohols while GB—A—1445956 and GB—A—1405127 disclose alkyl or hydroxy alkyl esters of, respectively, 16,17-dihydroapovincaminic and apovincaminic acids.

It has now been found that the methoxymethylester of apovincaminic acid, which is the object of the present invention, proved to have a considerably higher activity than the prior-art compounds, according to the results of pharmacological comparative experimentation (test of lethal effect of hypobaric hypoxia).

The compound of the above formula I which, for brevity purposes, will hereinafter referred to as MR 711, is prepared according to conventional procedures. For instance, one of these procedures comprises reacting substantially equimolecular proportions of an alkali metal salt of apovincamine, e.g. potassium apovincaminate, and a halomethyl-methy ether, e.g. bromomethyl-methyl-ether, in the presence of an organic solvent, e.g. methyl-ethyl-ketone. It is however intended that other conventional procedures suitable for preparing the compound of the above formula I fall within the scope of the present invention. The following example is given with the purpose of better illustrating the invention but in no way it must be construed as a limitation of the invention itself.

Example

A solution of 10.0 g (0.028 mole) of potassium apovincaminate in 100 ml of methylethyl-ketone was treated dropwise with 3.47 g (0.028 mole) of bromomethylmethyl ether at a temperature comprised between −5 and +5°C. The temperature was subsequently raised to 20°C and the reaction mixture was stirred until complete disappearance of the apovincaminic acid [Thin Layer Chromatography (TLC) monitorage; Merck plates; solvent system: ethyl acetate; visualization through U.V. light], then, after distilling off the solvent under reduced pressure, the obtained residue was dissolved in 100 ml of methylene chloride.

The methylene chloride solution was twice washed with water, dried over magnesium sulfate and evaporated *in vacuo,* thus obtaining an amorphous residue which was purified by column-chromatography through silica-gel, by employing ethyl acetate as the eluting system.

7.8 Grams of MR 8711, as white crystals, melting at 117°C, were obtained. The structure was confirmed by the I.R. and N.M.R. data.

Elemental analysis for $C_{22}H_{26}N_2O_3$

| | %C | %H | %N |
|---|---|---|---|
| Calc.: | 72.14 | 7.09 | 7.64 |
| Found: | 71.83 | 7.03 | 7.57 |

*I.R. (Nujol)* = 1720 $cm^{-1}$

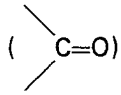

$^1H$—N.M.R. [$CDCl_3$, $\delta$ (in p.p.m.) from TMS (Tetra-MethylSilane, internal standard]: 3.5 (s, 3H, O—$CH_3$); 5.35 (s, 2H, O—$CH_2$—O); 6.1 (s, 1H, =CH—); (s=singlet).

As stated above the vincamine derivative according to the present invention possesses remarkable vasodilating and myorelaxing acrivities, coupled with a low acute toxicity. These favorable biological properties have been investigated by means of the following tests.

Acute toxicity

The experiments have been performed on mice, upon administration by intraperitoneal route of MR 711 and vincamine as the comparison compound.

The obtained $LD_{50}$ values, calculated according to Lichtfield and Wilcoxon (J. Pharm. Expt. Ther., *96*, 99, 1949) and reported in Table 1, clearly show that MR 711 is considerably less toxic than vincamine.

Vazodilating activity

The vasodilating activity has been investigated in rats, substantially following the kind paw perfusion technique according to Fostier e Smizk (J. Pharm. Expt. Ther, *89*, 256, 1947), and employing papaverine hydrochloride as the standard substance with acknowledged vasodilating action. Both substances were administered at a concentration of $5.10^{-5}$ g/ml. Also vincamine has been administered at the same dose. The obtained results, expressed as percent reduction of the perfusion pressure, have been reported in Table 2: they clearly demonstrate the remarkable vasodilating activity of MR 711, which is higher than that displayed by vincamine under the same experimental conditions.

Protective action on the mortality by hypobaric hypoxya

For this experiment mice have been placed into cages in which the internal pressure was progressively reduced until 190 mm Hg, i.e. a value incompatible with the survival.

MR 711 and vincamine have been both administered by intravenous route at a dosage of 25.6 mg/kg, and both have proven to be able to prolonge the survival time (see Table 3), but the effect displayed by MR 711 has been by far higher than that displayed by vincamine.

In vitro tests

The myorelaxing effect of MR 711 and vincamine has been investigated on the smooth musculature of the isolated ileum of rabbits, made hypertonic by sodium ions or serotonin.

Both MR 711 and vincamine have shown their efficacy in this test, but MR 711 has proven to be by far more active than the reference substance, in that its activity in inhibiting the sodium ions and serotonin induced contractions is respectively 46% and 70% higher than the corresponding activity displayed by vincamine.

TABLE 1

Acute toxicity in mice

| Compound | $LD_{50}$ mg/kg i.p. | Confidential limits (95%) |
|---|---|---|
| MR 711 | 580 | 540 – 620 |
| Vincamine | 195 | 142 – 210 |

TABLE 2

Vasodilating activity on the hind paw of rats

| Compound | Reduction of the perfusion pressure mm Hg | % |
|---|---|---|
| MR 711 | 38 ± 6.2 | 22.5 |
| Vincamine | 3.4 ± 2.8 | 2.3 |
| Papaverine hydrochloride | 62 ± 9.3 | 37.8 |

TABLE 3

Protective effect on the mortality by hypobaric hypoxya

| Compound | Survival time (seconds) | % Variation |
|---|---|---|
| Controls | 72.5 ± 3.5 | – |
| MR 711 | 262.9 ± 31.4 | +263 |
| Vincamine | 213.3 ± 37.9 | +194 |

The present invention also refers to all of the industrially applicable aspects connected with the use of the compound of formula I above as a vasodilating and myorelaxing agent. Thus, a further aspect of the present invention is represented by the pharmaceutical compositions containing predetermined amounts of the vincamine derivative of formula I above in admixture with one or more of the commonly employed pharmaceutically acceptable carriers.

As a matter of fact, the compound according to the invention may be administered by several routes, though the oral and parenteral one are the most preferred. For the oral administration, the compound of the invention is embodied into capsules, tablets, sugar coated tablets, lozenges, syrups, elixirs, dispersible powders, drops and the like, which contain the active ingredient in admixture with inert diluents such as, for instance, talc of lactose, lubricants such as, for instance, magnesium stearate, as well as dispersing agents, disintegrating agents, coloring and flavoring agents in order to provide an elegant and palatable composition. The pharmaceutical compositions suitable for the parenteral administration are formulated as known in the art. They usually contain the active ingredient in admixture with stabilizing and sterilizing agents. The active ingredient may be suspended or dissolved in pyrogen-free water.

These pharmaceutical compositions are suitable for being administered once or more time per day: they may contain from 2 to 60 mg of active principle. Typical, but not limitative, examples of pharmaceutical compositions are as follows:

—*capsules*, containing from 5 to 50 mg of active principle;

—*tablets*, containing from 5 to 50 mg of active principle;

—*vials* (for intramuscular or intravenous administration), containing from 5 to 15 mg of active principle;

—*drops*, containing from 1% to 5% by weight of active principle.

**Claims for the Contracting States: BE CH DE FR GB LI LU NL SE**

1. The compound 13a-ethyl-2,3,5,6,13a,13b-hexahydro-1H-indolo[3,2,1-de]pyrido[3,2,1-ij]-[1,5]naphthyridine-12-methoxymethyl carboxylate of the following formula (I)

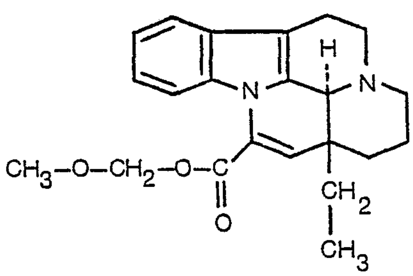

(I)

2. A process for preparing the compound of claim 1, which comprises reacting equimolecular proportions of an alkali metal salt of apovincamine and a halomethyl-methyl ether, in the presence of an organic solvent.

3. A process as defined in claim 2, wherein the alkali metal salt of apovincamine is potassium apovincaminate.

4. A process as defined in claim 2, wherein the halomethyl-methyl ether is bromomethyl-methyl ether.

5. A process as defined in claim 2, wherein the reaction solvent is methylethyl-ketone.

6. Pharmaceutical compositions having vasodilating and myorelaxing activity, which contain from 2 to 60 mg of the compound as defined in claim 1 in admixture with one or more pharmaceutical carriers.

7. Pharmaceutical compositions as defined in claim 6 suitable for oral or parenteral administration.

**Claims for the Contracting State: AT**

1. A process for preparing the compound 13a-ethyl-2,3,5,6,13a,13b-hexahydro-1H-indolo-[3,2,1-de]pyrido[3,2,1-ij]-[1,5]naphthyridine-12-methoxymethyl carboxylate of the following formula (I)

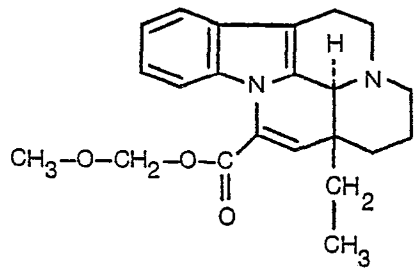

(I)

which comprises reacting equimolecular proportions of an alkali metal salt of apovincamine and a halomethyl-methyl ether, in the presence of an organic solvent.

2. A process as defined in claim 1, wherein the alkali metal salt of apovincamine is potassium apovincaminate.

3. A process as defined in claim 1, wherein the halomethyl-methyl ether is bromomethyl-methyl ether.

4. A process as defined in claim 1, wherein the reaction solvent is methylethyl-ketone.

**Revendications pour les Etats contractants: BE CH DE FR GB LI LU NL SE**

1. Le composé 13a-éthyl-2,3,5,6,13a,13b-hexahydro-1H-indolo[3,2,1-de]pyrido[3,2,1-ij]-[1,5]-naphthyridine-12-carboxylate de méthoxyméthyle de formule:

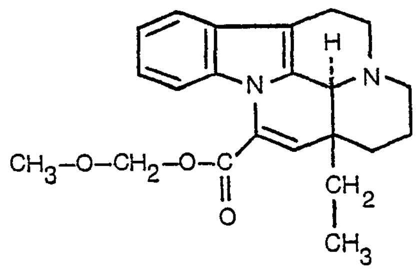

(I)

2. Procédé de préparation du composé selon la revendication 1, qui consiste à faire réagir des quantités équimolaires d'un sel de métal alcalin de l'apovincamine et d'un halométhyl-méthyl éther, en présence d'un solvant organique.

3. Procédé selon la revendication 2, dans lequel le sel de métal acalin de l'apovincamine est l'apovincaminate de potassium.

4. Procédé selon la revendication 2, dans lequel l'halométhyl-méthyl éther est le bromométhyl-méthyl éther.

5. Procédé selon la revendication 2, dans lequel le solvant de réaction est la méthyléthylcétone.

6. Compositions pharmaceutiques à activité vasodilatatrice et myorelaxante qui contient 2 à 60 mg du composé défini à la revendication 1, en mélange avec un ou plusieurs supports pharmaceutiques.

7. Compositions pharmaceutiques selon la revendication 6, appropriées à l'administration orale ou parentérale.

**Revendications pour l'Etat contractant: AT**

1. Procédé de préparation du composé 13a-éthyl-2,3,5,6,13a,13b-hexahydro-1H-indolo[3,2,1-de]pyrido[3,2,1-ij]-[1,5]-naphthyridine-12-carboxylate de méthoxyméthyle de formule:

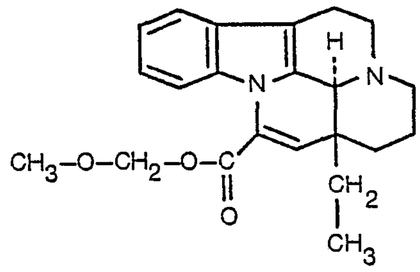

(I)

qui consiste à faire réagir des quantités équimolaires d'un sel de métal alcalin de l'apovincamine et d'un halométhyl-méthyl éther, en présence d'un solvant organique.

2. Procédé selon la revendication 1, dans lequel le sel de métal alcalin de l'apovincamine est l'apovincaminate de potassium.

3. Procédé selon la revendication 1, dans lequel l'halométhyl-méthyl éther est le bromométhyl-méthyl éther.

4. Procédé selon la revendication 1, dans lequel le solvant de réaction est la méthyléthylcétone.

**Patentansprüche für die Vertragsstaaten: BE CH DE FR GB LI LU NL SE**

1. Die Verbindung 13a-Äthyl-2,3,5,6,13a,13b-hexahydro-1H-indolo[3,2,1-de]pyrido[3,2,1-ij]-[1,5]-naphthyridin-12-methoxymethyl carboxylat der folgenden Formel (I)

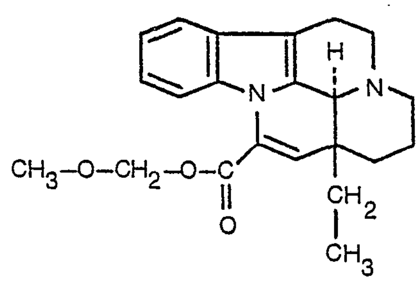

(I)

2. Verfahren zum Herstellen der Verbindung nach Anspruch 1, worin äquimolare Mengen eines Alkalimetallsalzes von Apovincamin und eines Halogenmethyl-methyläthers in Gegenwart eines organischen Lösungsmittels zur Reaktion gebracht werden.

3. Verfahren nach Anspruch 2, worin das Alkalimetallsalz von Apovincamin Kaliumapovincaminat ist.

4. Verfahren nach Anspruch 2, worin das Halogenmethyl-methyläther Brommethylmethyl-äther ist.

5. Verfahren nach Anspruch 2, worin das Reaktionslösungsmittel Methyläthyl-keton ist.

6. Pharmazeutische Mischung, die vasodilierende und muskelentspannende Wirksamkeit besitzt, welche von 2 bis 60 mg der Verbindung, wie in Anspruch 1 angegeben, in Beimischung mit einem oder mehreren pharmazeutischen Trägerstoffen enthält.

7. Pharmazeutische Mischung nach Anspurch 6, für orale oder parenterale Verabreichung.

**Patentansprüche für den Vertragsstaat: AT**

1. Verfahren zur Herstellung der Verbindung 13a-Äthyl-2,3,5,6,13a,13b-hexahydro-1H-indolo[3,2,1-de]pyrido[3,2,1-ij]-[1,5]-naphthyridin-12-methoxymethyl carboxylat der folgenden Formel (I)

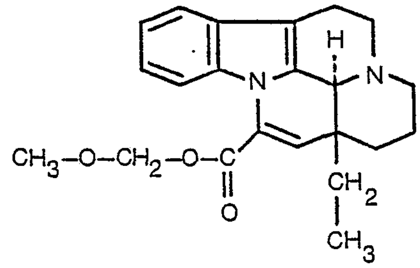

(I)

worin äquimolare Mengen eines Alkalimetallsalzes von Apovincamin und eines Halomethyl-methyläthers in Gegenwart eines organischen Lösungsmittels zur Reaktion gebracht werden.

2. Verfahren nach Anspruch 1, worin das Alkalisaltz von Apovincamin Kaliumapovincaminat ist.

3. Verfahren nach Anspruch 1, worin das Halomethyl-methyläther Brommethyl-methyläther ist.

4. Verfahren nach Anspruch 1, worin das aktionslösungsmittel Methyläthylketon ist.